# EUROPÄISCHE PATENTSCHRIFT

(11) **EP 2 190 308 B1**
(45) Veröffentlichungstag und Bekanntmachung des Hinweises auf die Patenterteilung: **29.08.2012**
(21) Anmeldenummer: 08804615.6
(22) Anmeldetag: 23.09.2008
(51) Int. Cl.: A23L 3/00, A23L 3/10, A23L 3/14, A61L 2/04, A61L 2/07

(54) **VORRICHTUNG ZUM STERILISIEREN VON QUADERFÖRMIGEN KARTON/KUNSTSTOFF-VERBUNDPACKUNGEN IN EINEM AUTOKLAVEN**
DEVICE FOR STERILIZING CUBOID CARTONS/PLASTIC COMBINATION PACKS IN AN AUTOCLAVE
DISPOSITIF DE STÉRILISATION D'EMBALLAGES COMPOSITES PARALLÉLÉPIPÉDIQUES EN CARTON ET MATIÈRE SYNTHÉTIQUE DANS UN AUTOCLAVE

(30) Priorität: 24.09.2007 DE 102007045720
(43) Veröffentlichungstag der Anmeldung: 02.06.2010
(73) Patentinhaber: SIG Technology AG, 8212 Neuhausen am Rheinfall (CH)
(72) Erfinder: WOLTERS, Michael, 52525 Heinsberg (DE); DINTELMANN, Thomas, 64380 Rossdorf (DE)
(74) Vertreter: Cohausz & Florack
(86) Internationale Anmeldenummer: PCT/EP2008/062700
(87) Internationale Veröffentlichungsnummer: WO 2009/040347

(56) Entgegenhaltungen:
- EP-A- 0 724 887
- EP-A- 1 382 353
- EP-A- 1 862 082
- WO-A-02/28721
- WO-A-98/16431
- WO-A-02/090206
- WO-A-03/103417
- FR-A- 2 290 249
- GB-A- 759 238
- GB-A- 762 335
- GB-A- 2 040 668
- JP-A- 9 028 772
- JP-A- 9 215 733
- JP-A- 10 033 643
- JP-A- 2001 231 520
- JP-A- 2002 101 862
- JP-A- 2003 319 768
- US-A- 5 705 127

## Beschreibung

Die Erfindung betrifft eine Vorrichtung zum Sterilisieren von quaderförmigen Karton/Kunststoff-Verbundpackungen in einem Autoklaven, wobei die aus Produkt und Verpackung bestehenden Packungen einer Wärmebehandlung bei einer bestimmten Temperatur über eine bestimmte Dauer unterzogen werden, wobei das Produkt aus einer Flüssigkeit und stückigen Anteilen besteht, wobei im Inneren des Autoklaven Einrichtungen zum rotatorischen Antrieb der Packungen um eine Drehachse vorgesehen sind, wobei eine Mehrzahl von in parallelen Reihen dicht benachbart angeordneten Packungen auf Tragböden angeordnet sind, und wobei eine Mehrzahl von mit Packungen bestückten Tragböden übereinander angeordnet und in ihrer gegenseitigen Lage fixiert sind.

Es ist seit langem bekannt, portioniert abgepackte Produkte wie beispielsweise Lebensmittel zur Haltbarmachung einem so genannten Autoklavierprozess zu unterziehen. Hierbei werden beispielsweise Konservendosen, in welche zuvor Lebensmittelprodukte abgepackt worden sind in einem Autoklaven für eine bestimmte Dauer einer bestimmten Temperatur ausgesetzt, um die im Produkt bzw. der Dose befindlichen Keime zuverlässig abzutöten. Konservendosen haben sich für den genannten Zweck insbesondere deshalb bewehrt, weil sie unempfindlich gegenüber den in einem Autoklaven herrschenden Bedingungen (hohe Temperatur, hohe Feuchte, hoher Druck) sind.

Werden Lebensmittel oder andere zu sterilisierende Produkte jedoch nicht in Konserven sondern in anderen Verpackungen auf den Markt gebracht so muss - mit relativ hohem Aufwand - zunächst die Verpackung sterilisiert werden, dann das Produkt unter sterilen Bedingungen in die Verpackung gefüllt werden und schließlich die Verpackung aseptisch geschlossen werden. Auf diese Weise hergestellte Karton/Kunststoff-Verbundpackungen sind in der Lage, Produkte wie beispielsweise Milch oder püriertes Gemüse über einen längeren Zeitraum aufzunehmen.

Aus der DE 102 33 095 A1 ist eine Vorrichtung bekannt, bei der mit Hilfe eines Rotationsautoklavprozesses zum Sterilisieren von Lebensmitteln Karton/Kunststoffverbundpackungen in einem Autoklaven während der Behandlung derart bewegt werden, dass eine ständige Durchmischung des Packungsinhaltes während des Autoklavierprozesses gewährleistet ist. Die hierfür verwendeten Mittel sind jedoch äußerst aufwendig, so dass sich dieser Prozess nicht kommerziell akzeptabel realisieren lässt.

Aus der EP 1 382 353 A ist eine Vorrichtung zum Sterilisieren von abgepackten Produkten nach dem Oberbegriff von Patentanspruch 1 bekannt.

Davon ausgehend liegt der vorliegenden Erfindung die Aufgabe zu Grunde, die eingangs genannte Vorrichtung so auszugestalten und weiterzubilden, dass eine zufrieden stellende Sterilisation einer größeren Anzahl von Packungen ermöglicht wird, ohne dass die Packungen verformt werden oder ihr Druckbild beschädigt wird.

Hinsichtlich der Vorrichtung mit den Merkmalen des Oberbegriffs von Anspruch 1 besteht die Lösung darin, dass zur Aufnahme der Packungen ein im Wesentlichen quaderförmiges Rahmengestell vorgesehen ist, welches eine Mehrzahl übereinander angeordneter tablettartiger Einsatzbleche zur Aufnahme einer Mehrzahl von in parallelen Reihen dicht hintereinander angeordneten und auf ihren Schmalseiten liegenden Packungen enthält und die Einsatzbleche so ausgebildet sind, dass zwischen dem Boden eines Einsatzbleches und den darunter befindlichen Packungsoberflächen des darunter angeordneten Einsatzbleches bei Beladetemperatur ein schmaler Luftspalt vorhanden ist.

Diese "Pakete" von ebenen Packungslagen lassen sich beispielsweise durch eine Verklemmung durch für sich bekannte pneumatische Einrichtungen in ihrer gegenseitigen Lage fixieren, damit sie im Rotationsautoklaven rotieren können, ohne dass es zu Lageänderungen einzelner zu behandelnder Packungen kommt. Zweckmäßigerweise befindet sich dazu auf der obersten Packungslage ein weiterer Tragboden, der gewissermaßen als "Deckel" wirkt. Die einzelnen Tragböden können darüber hinaus mit für sich bekannten Zentriereinrichtungen lagestabil im gewünschten parallelen Abstand zueinander fixiert werden.

Erfindungsgemäß ist zudem zur Aufnahme der Packung ein im Wesentlichen quaderförmiges Rahmengestell vorgesehen, welches eine Mehrzahl übereinander angeordneter tablettartiger Einsatzbleche zur Aufnahme einer Mehrzahl von in parallelen Reihen dicht hintereinander angeordneten und auf ihren Schmalseiten liegenden Packungen enthält.

Die Erfindung hat erkannt, dass eine Beschädigung des Druckbildes oder gar eine Verformung der Packung dadurch zuverlässig ausgeschlossen werden kann, dass die zu sterilisierenden Packungen im Inneren des Autoklaven mittels der speziell ausgebildeten Tragböden bzw. Einsatzbleche derart fixiert bzw. geführt werden, dass beim Rotieren einerseits eine gleichzeitige Durchmischung des Packungsinhaltes erfolgt und andererseits ein ausreichender Kontakt zwischen Heißdampf bzw. heißen Blechabschnitten und Packungsoberfläche gewährleistet ist.

Eine zufriedenstellende Sterilisation von Packungen aus kommerziell verfügbaren Packstoffen führt aufgrund der lokalen Durchnässung des Kartons zu Dellen, Beulen, und/oder Blasen sowie Druckbildbeschädigungen an der Packung. Aufgrund der hohen Temperaturen, hohen Feuchte und des hohen Drucks im Autoklaven wird die kommerziell verfügbare Verbundstruktur derart geschwächt, dass es zu unerwünschten Abdrücken der Packungsfixierelemente kommt. Teilweise können auch Delaminierungen des Packstoffmaterials auftreten. Ferner kann es bei der Entnahme von erweichten Packungen zu weiteren Beschädigungen kommen.

Der Rotationsprozess leidet systembedingt an der höheren Beanspruchung der Nähte bzw. Schnittkanten der Packung, so dass sich das unabhängig von der Drehrichtung und Anordnung der Packungen im Autoklaven zeitweise Auftreffen von teils flüssigen und teils gasförmigen Heiz- bzw. Kühlmedien, im Gegensatz zum statischen Prozess nicht verhindern lässt. Daher können konventionelle Packungen dieser erhöhten Beanspruchung nicht standhalten.

Hinsichtlich der verwendeten Packungen kann vorgesehen sein, dass der Karton zur Verminderung des Wassereintritts mittels Leim hydrophobiert ist und dass die eingesetzte Leimmenge zwischen 1 und 4 kg/t trockenen Faserstoffs liegt.

Gemäß einer weiteren Lehre der Erfindung in der Ausführung mit Rahmengestell ist vorgesehen, dass der Autoklav ein Dampf/Luft-Rotationsautoklav bzw. ein Berieselungs-Rotationsautoklav ist. Aufgrund des modularen und kompakten Aufbaus der erfindungsgemäßen Vorrichtung lassen sich ohne weiteres handelsübliche Rotationsautoklaven einsetzen.

Gemäß einer weiteren Ausgestaltung der Erfindung weist das Rahmengestell wenigstens einen aufklappbaren Deckel auf. Dies ist besonders zweckmäßig, da ein Deckel lediglich arretiert zu werden braucht, um die Einsatzbleche nach dem Beladen in ihrer Stellung so zu fixieren, dass sie beim Rotieren ihre gegenseitige Lage nicht verlieren. Zum Öffnen bzw. Entladen muss dann die Arretierung nur gelöst werden, um den Deckel aufzuklappen und die einzelnen Einsatzbleche entnehmen zu können. Eine Verschraubung zur Lagesicherung kann daher vollständig entfallen.

Zum leichteren Einbringen in den bzw. Ausbringen aus dem Autoklaven ist nach einer weiteren Ausgestaltung der Erfindung das Rahmengestell mit Rädern, Kufen, Ketten, oder Schienen oder dergleichen versehen, welche auf entsprechenden horizontalen Schienen, Führungen oder Rollen verfahrbar sind, um den Beschickungsprozess des Autoklaven zu vereinfachen.

Eine weitere Lehre der Erfindung sieht vor, dass jedes Einsatzblech einen umlaufenden Rahmen und zwischen den Packungsreihen verlaufende Stegbleche aufweist. Auf diese Weise werden die Packungen großflächig in ihrer Lage fixiert und vor einer Verformung geschont. Bevorzugt sind der Rahmen und/oder die Stegbleche hohl ausgeführt und weisen Ausnehmungen auf, um eine gute Zirkulation des Sterilisiermediums im Inneren des Autoklaven zu ermöglichen. Die Ausnehmungen sind dabei in weiterer Ausgestaltung der Erfindung so angeordnet, dass die Packungen an den sich berührenden Kontaktflächen nicht im Bereich von Ausnehmungen liegen. Auch hierdurch wird eine optimale Schonung der Packungen vor Verformung erreicht.

Die Erfindung hat darüber hinaus erkannt, dass es zweckmäßig ist, wenn der Boden jedes Einsatzbleches keine Ausnehmungen oder Löcher aufweist. Ferner kann der Boden jedes Einsatzbleches als Hohlboden ausgeführt sein, so dass durch ihn zur besseren Temperierung Luft bzw. Dampf strömen können.

Erfindungsgemäß sind die Einsatzbleche so ausgebildet, dass zwischen dem Boden eines Einsatzbleches und den darunter befindlichen Packungsoberflächen bei Beladetemperatur ein schmaler Luftspalt vorhanden ist. Die Breite des Luftspaltes kann - bei Beladetemperatur - 0,5 bis 10 mm, vorzugsweise 3 bis 5 mm betragen. Der Gegendruck im Rotationsautoklaven wird aber einer Temperatur von oberhalb 100°C so geführt, dass durch die thermische Ausdehnung der Verpackung in Verbindung mit der Ausdehnung des Kopfraums bzw. der Ausdehnung der Gasblase, die Verpackung zwischen den parallelen Flächen der Autoklavtrays verklemmt bzw. fixiert wird und so eine Beschädigung der Verpackung vermieden wird.

Es hat sich ferner gezeigt, dass ein gutes Sterilisierergebnis dann erreicht wird, wenn die Längsachse der Packungsreihen parallel zur Rotationsachse des Autoklaven liegt. Eine technisch äußerst aufwendige und raumgreifende Rotation der zu sterilisierenden Packungen um eine nicht zu den Packungskanten parallel angeordnete Raumachse, wie beim gattungsgemäßen Stand der Technik, kann somit entfallen.

Es kann vorgesehen sein, dass die Dichte des verwendeten Rohkartons im Bereich von 680 bis 860 kg/m³ liegt. Vorzugsweise beträgt der Mahlgrad der für die Packung verwendeten Kartonfasern 15 bis 35 SR. Ein solches Material kann den im Autoklaven herrschenden extremen Bedingungen gut standhalten.

Die Oberflächen der Packungen können hydrophob bedruckt sein und/oder der Druck kann durch eine transparente Kunststoffschicht geschützt sein. Eine Beschädigung des Druckbildes durch Abrieb und Abplatzen oder Verfärben ist mit dieser Ausgestaltung zuverlässig ausgeschlossen.

Eine besonders gute Sterilisation lässt sich dann erreichen, wenn die Füllmenge der verwendeten Packungen so gewählt ist, dass in ihrem Inneren eine Gasblase von 2 bis 30 % des Nennvolumens der Packung vorhanden ist. Dadurch und die im Produkt befindlichen stückigen Anteile findet beim Rotieren im Autoklaven eine gleichmäßige und ständige Durchmischung des Packungsinhaltes statt. Dies führt zu deutlich kürzeren Behandlungszeiten, was eine weitere Schonung der verwendeten Packungen nach sich zieht. Als Gas lässt sich in bekannter Weise beispielsweise Stickstoff einsetzen

Die Schnittkanten der Packungen können zusätzlich verpresst sein. Während die Packungsoberflächen durch die PP-Beschichtung und/oder die hydrophobe Bedruckung relativ gut vor Eindringen von Feuchtigkeit geschützt sind, sind die "offenen" Schnittkanten im Bereich der Nähte besonders gefährdet. Aus diesem Grunde verhindert eine zusätzliche Verpressung das Eindringen von Dampf oder Feuchtigkeit in das Verbundmaterial.

Zum Abfangen des Sauerstoffanteils im Karton/Kunstoff - Verbundmaterial können Scavenger eingesetzt werden. Die Verwendung solcher "Sauerstofffänger" in Packstoffmaterial ist für sich bereits bekannt.

Die Erfindung wird nachfolgend anhand einer lediglich ein Ausführungsbeispiel darstellenden Zeichnung näher erläutert. In der Zeichnung zeigen
- Fig. 1:: eine Ausführungsform der erfindungsgemäßen Vorrichtung in Stirnansicht in einem nur schematisch angedeuteten Autoklaven,
- Fig. 2:: die erfindungsgemäße Vorrichtung aus Fig. 1 in Seitenansicht,
- Fig. 3:: die erfindungsgemäße Vorrichtung aus Fig. 1 in Draufsicht, jedoch mit geöffnetem Deckel,
- Fig. 4:: einen vergrößerten Querschnitt durch oberen Teil der erfindungsgemäßen Vorrichtung, in Richtung der Pfeile IV-IV in Fig. 2,
- Fig. 5:: einen Eckausschnitt eines leeren Einsatzbleches in perspektivischer Darstellung und
- Fig. 6:: den Eckausschnitt aus Fig. 5 mit einer eingesetzten Packung.

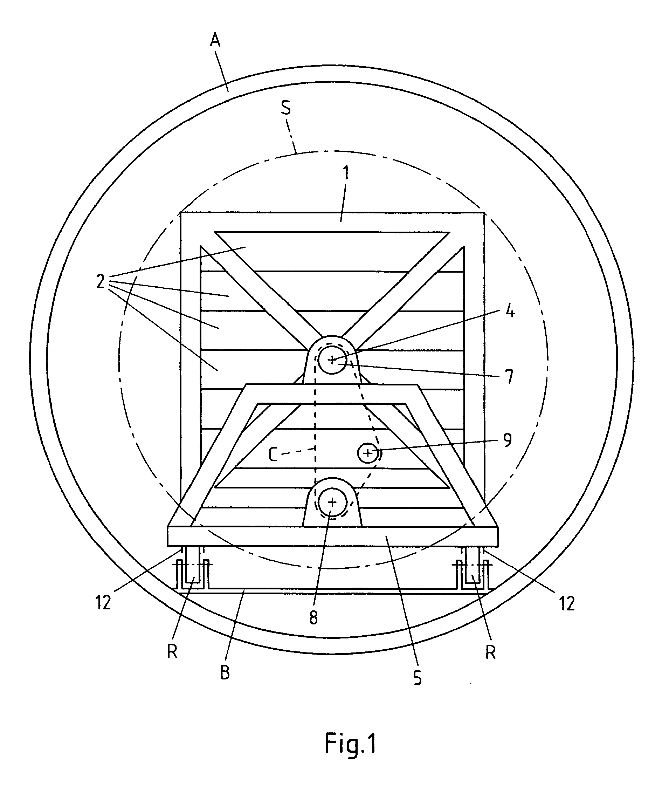

Die erfindungsgemäße Vorrichtung zum Sterilisieren von quaderförmigen Kartons/Kunststoff-Verbundpackungen besteht, wie aus den Fig. 1 bis 3 ersichtlich, zunächst aus einem Rahmengestell 1, in welches eine Mehrzahl von Einsatzblechen 2 eingesetzt sind. Damit die Einsatzbleche 2 beim Rotieren des Rahmengestells 1 nicht aus diesem herausfallen, besitzt das Rahmengestell 1 einen arretierbaren Deckel 3.

Wie bereits erwähnt, umfasst die Erfindung jedoch auch "rahmenlose" Ausführungen, bei denen die in mehreren Ebenen übereinander angeordneten Packungen zur Fixierung gegeneinander verklemmt werden, doch soll hier beispielhaft eine Vorrichtung mit einem Rahmengestell 1 erläutert werden.

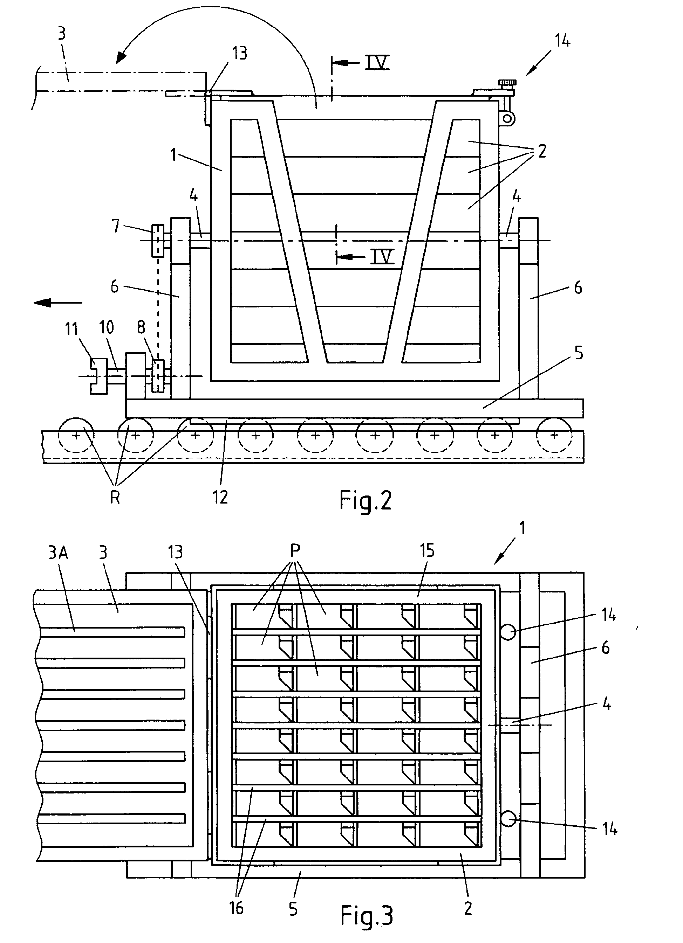

Das Rahmengestell 1 ist über Achsen 4 in auf einem Grundrahmen 5 befindlichen seitlichen Stützen 6 drehbar gelagert. Zum Aufbringen des notwendigen Drehmoments ist die in der Fig. 2 links angeordnete Drehachse 4 endseitig mit einem Kettenrad 7 versehen, welches über eine gestrichelt dargestellte Kette C mit einem weiteren Kettenrad 8 sowie einem nur in Fig. 1 angedeuteten Spannrad 9 mit einer Antriebswelle 10 verbunden ist, welche endseitig eine Klauenkupplung 11 aufweist, die beim Hineinfahren in den Autoklaven A in Richtung des nicht näher bezeichneten Pfeils mit einer entsprechenden Kupplung eines Antriebsmotors (nicht dargestellt) in Wirkverbindung tritt.

Zum Erleichtern des Beschickungsvorganges verfügt der Grundrahmen 5 an seiner Unterseite über Führungsschienen 12, die zur Führung entlang an einer Basis B des Autoklaven A freidrehend befestigten Rollen R dienen, wie dies aus den Fig. 1 und 2 deutlich hervorgeht.

Aus Fig. 2 ist ferner zu entnehmen, dass der Deckel 3 mittels eines Gelenks 13 am Rahmengestell 1 befestigt ist und mit Hilfe von zwei Verschlüssen 14 in seiner geschlossenen Stellung arretiert werden kann. Das geschlossene Rahmengestell 1 lässt sich dann im Inneren des Autoklaven A um die Rotationsachse 4 drehen; der zugehörige Schwenkkreis S ist in Fig. 1 strichpunktiert dargestellt.

Aus Fig. 3 geht nun hervor, dass jedes Einsatzblech 2 von denen nur das oberste bei aufgeklapptem Deckel 3 sichtbar ist, über einen umlaufenden Rahmen 15 verfügt, der die seitliche Begrenzung einer Vielzahl von Packungen P bildet. Damit die Packungen P in gleichmäßigen Längsreihen hintereinander und auf ihren Schmalseiten liegend innerhalb des Einsatzbleches 2 angeordnet werden können, verfügt jedes Einsatzblech 2 über die gesamte Länge des Einsatzbleches 2 verlaufende Stegbleche 16, die eine seitliche Begrenzung für die benachbarten Packungsreihen darstellen.

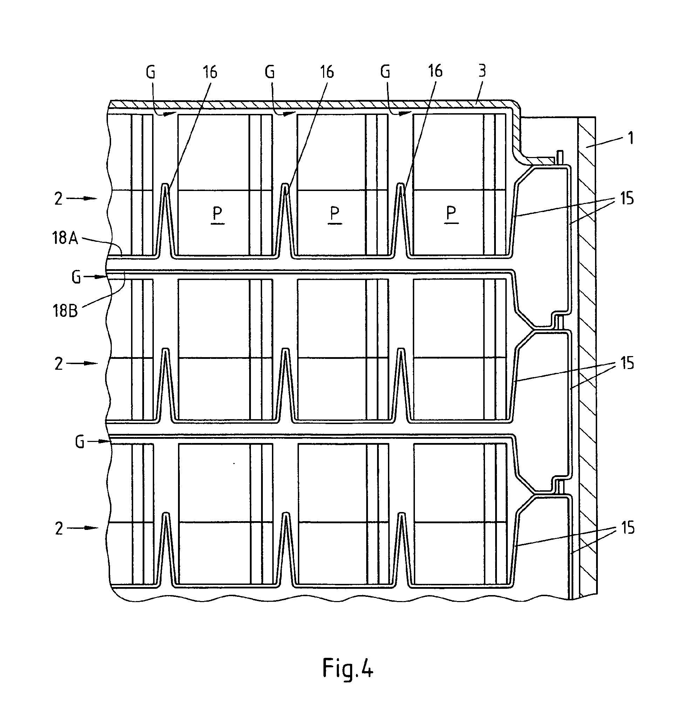

Der genaue Aufbau der erfindungsgemäßen Einsatzbleche 2 lässt sich aus Fig. 4 entnehmen, in der der obere Teil eines Paketes von Einsatzblechen 2 aus Fig. 2 im Querschnitt in Richtung der Pfeile IV-IV gezeigt ist. Hier ist zunächst zu erkennen, dass der Rahmen 15 der Einsatzbleche 2 hohl ausgebildet ist und so weit hinunter reicht, dass er gewissermaßen gleichzeitig einen oberen "Deckel" für die unter ihm angeordneten Packungen P darstellt. Der Deckel 3 besitzt daher an seiner Unterseite die gleiche Form. Ferner ist erkennbar, dass die Stegbleche 16 so angeordnet sind, dass sie nach oben spitz zulaufen, unten jedoch jeweils eine Packungsbreite definieren. Ein solcher Aufbau ermöglicht eine leichte seitliche Bewegung der Packungen P beim Drehvorgang im Autoklaven A.

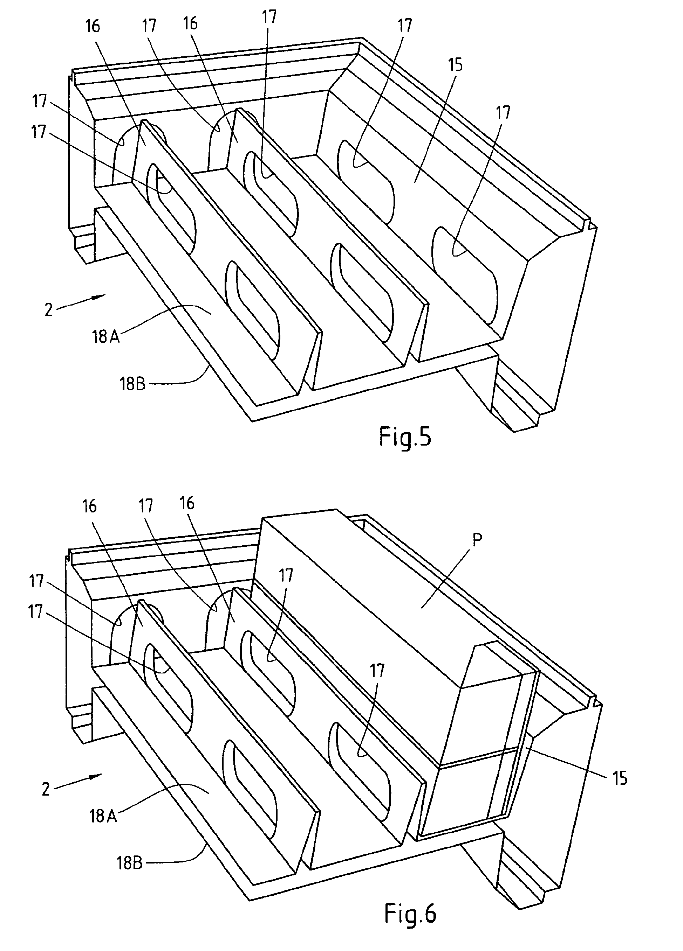

Damit sich nun das Sterilisiermedium optimal auch im Bereich der Einsatzbleche 2 verteilen kann, weisen sowohl der Rahmen 15 als auch die Stegbleche 16 Ausnehmungen 17 auf. Der Boden 18 der Einsatzbleche 2 ist als Hohlboden ausgeführt, d. h. hier besteht aus einem oberen Boden 18A und einem unteren Boden 18B wie in den perspektivischen Darstellungen gemäß Fig. 5 und Fig. 6 deutlich gezeigt. Diese Ausbildung hat den Vorteil, dass die geschlossenen Böden 18A, welche nicht mit Ausnehmungen oder Löchern versehen sind, eine optimale Abstützung der schmalen Längsseiten der Packungen P darstellen. Zur besseren Übersicht ist in Fig. 6 eine in das gezeigte Einsatzblech 2 eingesetzte Packung P dargestellt. Dadurch, dass oberhalb des gezeigten Einsatzbleches 2 ein weiteres Einsatzblech 2 bzw. der Deckel 3, welcher ebenfalls mit Schlitzen 3A versehen ist, die Packungsreihen nach oben hin begrenzen, erfolgt auch eine entsprechende Abstützung bei der Rotation des Rahmengestells 1. Die Schlitze 3A sind, wie aus Fig. 3 hervorgeht, dazu genau oberhalb der Stegbleche 16 angeordnet.

Die Höhe der Einsatzbleche 2 ist dabei so gewählt, dass zwischen dem Boden 18B eines Einsatzbleches 2 und den darunter befindlichen Oberflächen der Packungen P bei Beladetemperatur ein schmaler Luftspalt G vorhanden ist, wie aus Fig. 4 hervorgeht. Bevorzugt beträgt die Breite des Spaltes (G) bei Beladetemperatur 0,5 bis 10 mm.

## Patentansprüche

1. Vorrichtung zum Sterilisieren von quaderförmigen Karton/Kunststoff-Verbundpackungen in einem Autoklaven, wobei die aus Produkt und Verpackung bestehenden Packungen einer Wärmebehandlung bei einer bestimmten Temperatur über eine bestimmte Dauer unterzogen werden, wobei das Produkt aus einer Flüssigkeit und stückigen Anteilen besteht, wobei im Inneren des Autoklaven Einrichtungen zum rotatorischen Antrieb der Packungen um eine Drehachse vorgesehen sind, wobei eine Mehrzahl von in parallelen Reihen dicht benachbart angeordneten Packungen (P) auf Tragböden angeordnet sind, und wobei eine Mehrzahl von mit Packungen (P) bestückten Tragböden übereinander angeordnet und in ihrer gegenseitigen Lage fixiert sind,
**dadurch gekennzeichnet, dass** zur Aufnahme der Packungen ein im Wesentlichen quaderförmiges Rahmengestell (1) vorgesehen ist, welches eine Mehrzahl übereinander angeordneter tablettartiger Einsatzbleche (2) zur Aufnahme einer Mehrzahl von in parallelen Reihen dicht hintereinander angeordneten und auf ihren Schmalseiten liegenden Packungen (P) enthält und die Einsatzbleche (2) so ausgebildet sind, dass zwischen dem Boden (18B) eines Einsatzbleches (2) und den darunter befindlichen Packungsoberflächen des darunter angeordneten Einsatzbleches (2) bei Beladetemperatur ein schmaler Luftspalt (G) vorhanden ist.

2. Vorrichtung nach Anspruch 1,
**dadurch gekennzeichnet, dass** der Autoklav (A) ein Dampf/Luft-Rotationsautoklav ist.

3. Vorrichtung nach Anspruch 1,
**dadurch gekennzeichnet, dass** der Autoklav (A) ein Berieselungs-Rotationsautoklav ist.

4. Vorrichtung nach einem der Ansprüche 1 bis 3,
**dadurch gekennzeichnet, dass** das Rahmengestell (1) wenigstens einen aufklappbaren Deckel (3) aufweist.

5. Vorrichtung nach einem der Ansprüche 1 bis 4,
**dadurch gekennzeichnet, dass** das Rahmengestell (1) Räder, Kufen, Ketten und/oder Schienen (12) zum leichteren Ein- bzw. Ausbringen in den bzw. aus dem Autoklaven (A) aufweist.

6. Vorrichtung nach einem der Ansprüche 1 bis 5,
**dadurch gekennzeichnet, dass** jedes Einsatzblech (2) einen umlaufenden Rahmen (15) und zwischen den Packungsseiten verlaufende Stegbleche (16) aufweist.

7. Vorrichtung nach Anspruch 6,
**dadurch gekennzeichnet, dass** der Rahmen (15) und/oder die Stegbleche (16) hohl ausgebildet sind und Ausnehmungen (17) aufweisen.

8. Vorrichtung nach Anspruch 7,
**dadurch gekennzeichnet, dass** die Ausnehmungen (17) so angeordnet sind, dass die Packungen (P) an den sich berührenden Kontaktflächen nicht im Bereich von Ausnehmungen (17) liegen.

9. Vorrichtung nach einem der Ansprüche 1 bis 8,
**dadurch gekennzeichnet, dass** wenigstens im Kontaktbereich mit den Packungen (P) der Boden (18) jedes Einsatzbleches (2) keine Ausnehmungen oder Löcher aufweist.

10. Vorrichtung nach Anspruch 9,
**dadurch gekennzeichnet, dass** der Boden (18) jedes Einsatzbleches (2) als Hohlboden ausgeführt ist, so dass zwischen dem oberen (18A) und unteren Blech (18B) Luft bzw. Dampf strömen kann.

11. Vorrichtung nach einem der Ansprüche 1 bis 10,
**dadurch gekennzeichnet, dass** die Breite des Luftspaltes (G) 0,5 bis 10 mm beträgt.

12. Vorrichtung nach Anspruch 11,
**dadurch gekennzeichnet, dass** die Breite des Luftspaltes (G) bei Beladetemperatur 3 bis 5 mm beträgt.

## Claims

1. Device for sterilising cuboidal combined cardboard/plastics material packs in an autoclave, wherein the packs consisting of product and wrapping are subjected to a heat treatment at a specific temperature for a specific period of time, the product consisting of a liquid and fragmentary portions, means being provided inside the autoclave for driving the packs in a rotating manner about an axis of rotation, a plurality of packs (P), arranged closely next to one another in parallel rows, being arranged on supporting bases and a plurality of supporting bases provided with packs (P) being arranged one above another and being fixed in their mutual position, **characterised in that** for receiving the packs, a substantially cuboidal frame (1) is provided which contains a plurality of superimposed tray-like insertion sheets (2) for receiving a plurality of packs (P) which are arranged in parallel rows closely behind one another, lying on their narrow sides, and the insertion sheets (2) are configured such that there is a narrow air gap (G) at the loading temperature between the base (18B) of one insertion sheet (2) and the pack surfaces, located thereunder, of the insertion sheet (2) arranged thereunder.

2. Device according to claim 1, **characterised in that** the autoclave (A) is a steam/air rotary autoclave.

3. Device according to claim 1, **characterised in that** the autoclave (A) is a sprinkling rotary autoclave.

4. Device according to any one of claims 1 to 3, **characterised in that** the frame (1) has at least one hinged cover (3).

5. Device according to any one of claims 1 to 4, **characterised in that** the frame (1) has wheels, rockers, chains and/or rails (12) to facilitate its insertion into and removal from the autoclave (A).

6. Device according to any one of claims 1 to 5, **characterised in that** each insertion sheet (2) has an encompassing frame (15) and web plates (16) running between the sides of the packs.

7. Device according to claim 6, **characterised in that** the frame (15) and/or the web plates (16) are hollow and have recesses (17).

8. Device according to claim 7, **characterised in that** the recesses (17) are arranged such that the packs (P) lie on the mutually contacting contact surfaces not in the region of recesses (17).

9. Device according to any one of claims 1 to 8, **characterised in that** the base (18) of each insertion sheet (2) does not have any recesses or holes at least in the contact area with the packs (P).

10. Device according to claim 9, **characterised in that** the base (18) of each insertion sheet (2) is configured as a hollow base so that air or steam can flow between the upper sheet (18A) and the lower sheet (18B).

11. Device according to any one of claims 1 to 10, **characterised in that** the width of the air gap (G) is 0.5 to 10 mm.

12. Device according to claim 11, **characterised in that** the width of the air gap (G) is 3 to 5 mm at the loading temperature.

## Revendications

1. Dispositif pour stériliser dans un autoclave des briques parallélépipédiques composites en carton / matière synthétique, sachant que les briques, qui consistent en produit et emballage, sont soumises à un traitement à chaud, à une température et pendant une durée déterminées, le produit consistant en un liquide et en morceaux, sachant qu'à l'intérieur de l'autoclave sont prévus de dispositifs pour l'entraînement en rotation des briques autour d'un axe de rotation, une pluralité de briques (P), disposées en rangées parallèles, à proximité les uns des autres, étant posée sur des supports et plusieurs supports garnis de briques (P) étant disposés les uns au-dessus des autres et fixés dans leur position relative,
**caractérisé en ce que**,
pour la réception des briques, est prévu un cadre (1) de forme sensiblement parallélépipédique, qui comprend une pluralité d'éléments (2) en forme de plateaux, qui, disposés les uns au-dessus des autres, sont destinés à recevoir une pluralité de briques (P), disposées étroitement les uns derrière les autres, en rangées parallèles, en étant posées sur leurs côtés étroits, et les éléments (2) étant conçus de sorte qu'une fente d'aération (G) existe, à la température de chargement, entre le fond (18B) d'un élément (2) et la face supérieure des briques sous-jacentes, de l'élément (2) sous-jacent.

2. Dispositif selon la revendication 1, **caractérisé en ce que** l'autoclave (A) est un autoclave rotatif à vapeur / air.

3. Dispositif selon la revendication 1, **caractérisé en ce que** l'autoclave (A) est un autoclave rotatif à ruissellement.

4. Dispositif selon l'une des revendications 1 à 3, **caractérisé en ce que** le cadre (1) est doté d'au moins un couvercle (3) relevable.

5. Dispositif selon l'une des revendications 1 à 4, **caractérisé en ce que** le cadre (1) est doté de roues, de patins, de chaînes et / ou de rails (12) pour faciliter le chargement, respectivement le déchargement de l'autoclave (A).

6. Dispositif selon l'une des revendications 1 à 5, **caractérisé en ce que** chaque élément (2) est doté d'un cadre (15) périphérique et d'entretoises (16), qui s'étendent entre les côtés des briques.

7. Dispositif selon la revendication 6, **caractérisé en ce que** le cadre (15) et / ou les entretoises (16) sont creux et sont dotés d'évidements (17).

8. Dispositif selon la revendication 7, **caractérisé en ce que** les évidements (17) sont disposés de sorte que les briques (P), là où les faces de contact se touchent, ne se trouvent pas dans la région des évidements (17).

9. Dispositif selon les revendications 1 à 8, **caractérisé en ce que** le fond (18) de chaque élément (2) ne présente aucun évidement ou trou, au moins dans la zone de contact avec les briques (P).

10. Dispositif selon la revendication 9, **caractérisé en ce que** le fond (18) de chaque élément (2) est réalisé en tant que fond creux, de sorte que l'air, respectivement la vapeur puisse circuler entre la tôle supérieure (18A) et la tôle inférieure (18B).

11. Dispositif selon l'une des revendications 1 à 10, **caractérisé en ce que** la largeur de la fente d'aération (G) est de 0,5 à 10 mm.

12. Dispositif selon la revendication 11, **caractérisé en ce que** la largeur de la fente (G) est de 3 à 5 mm à la température de chargement.
